# EUROPEAN PATENT APPLICATION

(11) **EP 2 805 708 A1**
(43) Date of publication of application: **26.11.2014**
(21) Application number: 13168674.3
(22) Date of filing: 22.05.2013
(51) Int. Cl.: A61K 9/00, A61K 39/00, A61K 9/16, A61K 9/50

(54) **Methods to produce particles comprising therapeutic proteins**

(71) Applicant: UCB Pharma, S.A., 1070 Brussels (BE)
(72) Inventor: The designation of the inventor has not yet been filed
(74) Representative: UCB Intellectual Property

(57) **Abstract**

The present invention relates to methods for producing a particle comprising a polymer matrix and a protein.

## Description

### FIELD OF THE INVENTION

The present invention is in the field of pharmaceutical formulations. More specifically, it relates to methods for producing a microparticle comprising a therapeutic protein, such as an antibody.

### BACKGROUND OF THE INVENTION

Targeted and/or controlled release of molecules for therapeutic, diagnostic or preventive purposes such as vaccination is being widely explored as it offers potential benefits over conventional administration of therapeutics, diagnostics or vaccines. Targeted and/or controlled release of macromolecules, such as proteins, e.g. antibodies or polynucleotides represents a particular challenge in art.

Frequently proteins useful for therapeutic, diagnostic or preventive applications (e.g. vaccination) in animals or humans such as antibodies have to be administered in high doses to be effective. Vaccines, antibodies or other therapeutic or diagnostic proteins generally have to be administered parenterally to animals or humans. Commonly such proteins need to be injected, e.g. intravenously, subcutaneously or intramuscularly. Depot formulations that are released in a controlled way over prolonged periods such as days, weeks or months are thus required to avoid frequent injections and increase compliance with the required administration scheme.

Pharmaceutical formulations of approved therapeutic antibodies and other protein therapeutics typically are not suitable for controlled release of proteins such as antibodies. Controlled release formulations of antibodies or other therapeutic proteins are desired in order to allow for less frequent administration thereby reducing the need for injections and improving patient compliance. There is thus a need in the art to provide pharmaceutical formulations effecting controlled release of antibodies or other protein therapeutics.

One way to achieve targeted and controlled release of a molecule *in vivo* is through the use of particles, such as nanoparticles or microparticles. These particles can be administered through different routes, including oral and parenteral routes. Particles can e.g. be inhaled or injected, e.g. intravenously, subcutaneously or intramuscularly.

Particles that are useful for *in vivo* applications in animals or humans should be biodegradable and biocompatible. In the last two decades, synthetic biodegradable polymers have been used as carrier or in micro- or nanoparticles to deliver small molecule drugs [1]. Thermoplastic aliphatic poly(esters), such as poly-lactide [PLA], poly-glycolide [PGA], and especially poly(D,L-lactide-co-glycolide) [PLGA] have generated tremendous interest due to their excellent biocompatibility and biodegradability. Various polymeric drug delivery systems such as particles, microcapsules, nanoparticles, pellets, implants, and film have been produced using these polymers for the delivery of a variety of therapeutic drugs. They have also been approved by the FDA for drug delivery use.

PLA or PLGA-based particles can also be further modified, e.g. through the attachment of a targeting moiety such as poly(ethyleneglycol) [PEG] or a protein. One of the main limitations of this kind of particles is nonspecific adsorption of plasma proteins on microparticles. The adsorption of plasma proteins is believed to be a key factor in explaining organ distribution of microparticles, for example the presence of specific proteins is known to promote the ingestion by some cells via specific or unspecific interactions with cell membrane receptors. Therefore different modification strategies have been pursued to suppress this effect. The modification of PLGA particles with PEG is an important and well-known approach to reduce protein adsorption. Another option has been introducing functional groups on the PLGA microparticles via functionalized poly(L-lysine)-g-poly(ethylene glycol) (PLL-g-PEG), thus producing PLGA microparticles with a strongly decreased protein adsorption [2].

Another underlying cause for particle modification is targeting said particles to a desired tissue or cell type within the body in order to achieve the desired therapeutic effect. Specific delivery of the active ingredient to the desired target cells not only allows for increased efficiency, but also for a reduction in side-effects stemming from unwanted effects of the active principle on different tissues. This may be achieved using different targeting moieties such as antibodies, fragments thereof or receptor targeting peptides. For example, both antibodies and receptor targeting peptides have been shown to effectively target PLGA particles preferentially to dendritic cells after subcutaneous injection [3].

Advantages of polymer matrix based particles, in particular particles based on PLA, PGA or PLGA, over conventional drug delivery systems include extended releases of up to days, weeks or months, in addition to their biocompatibility and biodegradability. It has been noted, however, that the use of these polymer matrixes, is problematic in connection with proteins such as antibodies as the polymer matrix seems to have a negative effect on protein stability during preparation and storage, primarily due to the acid-catalyzed nature of its degradation. In addition, processing conditions used in the manufacture of the polymer matrix drug delivery vehicles have detrimental effects on protein secondary structure [4].

The generation of particles based on polymer matrices such as PLA, PGA or PLGA, requires multiple steps and involves multiple parameters. Polymer matrix-based particles comprising proteins, e.g. antibodies, are particularly challenging to produce due to the complexity and inherent instability of proteins and large proteins such as antibodies. Proteins and antibodies are prone to become inactive during the process of forming a polymer matrix-based particle.

Several methods have been used to produce PLGA-based particles. The most common method employs the emulsification-solvent evaporation method. In this method in the case of hydrophobic drugs both the polymer and the active molecule (e.g. a preventive, prognostic, diagnostic or therapeutic molecule)are dissolved in an organic solvent, such as methylene chloride, to form an emulsion oil. An emulsion oil (o) in water (w), i.e. o/w, is then prepared by subsequently adding water and an emulsifier such as polyvinyl alcohol (PVA) or polyvinyl pyrrolidone (PVP) to the polymer solution. The particles are induced by sonication or homogenization, and the solvent is then evaporated or then extracted in order to produce the particles. In cases of hydrophilic drugs such as proteins, the polymer is dissolved in an organic solvent and the active molecule is dissolved in an aqueous phase, from which a first water-oil emulsion is prepared. Next, water and an emulsifier is added to generate a double water-in oil-in water emulsion (w/o/w). From this second emulsion particles are then induced and solvent later evaporated or extracted to yield the particles.

Protein adsorption and denaturation at the water/solvent interfaces is one of the major factors for decreased protein bioactivity occurring during the microencapsulation process. To avoid protein denaturation which mainly occurs during formation of water-in-oil (w/o) emulsion in the water-in-oil-in-water (w/o/w) method, a solid-in-oil-in-water (s/o/w) method has been developed [5]. Indeed, in the solid state, proteins are believed to maintain their bioactivity by drastically reducing conformational mobility. In the s/o/w method, the polymer is dissolved in an organic solvent, in which solid protein particles are dispersed to generate the primary solid-in-oil (s/o) suspension. This suspension is then added to an aqueous phase containing an emulsifier such as PVA or PVP to form the s/o/w emulsion. The resultant emulsion is then maintained under stirring to allow both the extraction and the evaporation of the organic solvent and subsequent recovery of the particles.

One of the major issues in the s/o/w method, is protein particle micronization, i.e. reducing the average diameter of these solid particles, with micronization methods including lyophilization, spray-drying, and spray-freeze-drying [6] . Lyophilization, also known as freeze-drying is a dehydration process which works by freezing the material and then reducing the surrounding pressure to allow the frozen water in the material to sublimate directly from the solid phase to the gas phase. On the other hand, spray drying is a method of producing a dry powder from a liquid or slurry by rapidly drying with a hot gas. Air is the heated drying medium; however, if the liquid is a flammable solvent such as ethanol or the product is oxygen-sensitive then nitrogen is used. Lastly, spray-freeze-drying briefly, consists of the atomization of a liquid solution into a cryogenic gas or liquid with instant freezing of the generated droplets followed by sublimation of the ice at low temperature and pressure during freeze-drying.

In fact, it is spray-drying that is generally used to produce protein microparticles, although this method has problems such as low recovery and the risk of protein denaturation and agglomeration due to heat and physical stress

During this process, high protein loading and high encapsulation efficiency are critical parameters in view of the high cost of therapeutic proteins in general and antibodies in particular. The encapsulation efficiency (EE%) refers to the amount of active molecules detected in the microparticles compared to the amount of active molecules initially introduced into the organic phase. Whereas protein loading refers to the amount of active molecule present in the microparticle, generally expressed as a weight ratio.

Moreover, for particular applications such as administration by injection, the mean diameter of injectable particles should be small enough to be injected. Usually, 22-25 gauge needles (inner diameters of 394-241 µm) are used for intravenous infusion as well as intramuscular and subcutaneous injections. Therefore, particles characterized by a mean diameter lower than 250 µm, ideally less than 125 µm, are considered to be suitable for administration to individuals. The particle size and size distribution are also important factors in the protein release rate as the total surface area for protein delivery depends on the particle size [7].

There is a need in the art to provide improved methods for producing polymer matrix-based particles comprising antibodies or other therapeutic, diagnostic or preventive proteins. There is a need in the art for improved polymer matrix-based particles comprising antibodies or other therapeutic, diagnostic or preventive protein for controlled release. There is a need in the art to provide improved methods for producing polymer matrix-based particles with increased encapsulation efficiency in order to be able produce such particles at commercially viable costs.

### SUMMARY OF THE INVENTION

It is an object of the present invention to provide a method for producing a particle comprising a polymer matrix and a protein.

In one embodiment the invention provides a method for producing a particle comprising a polymer matrix and a protein comprising the steps of solidifying the protein from a solution comprising the protein, combining the solidified protein with a solvent comprising the polymer matrix, combining the solvent comprising the polymer matrix and the protein with an emulsifier to stabilize the emulsion in water, agitating the solvent comprising the polymer matrix, the protein and the emulsifier to form an emulsion, combining the emulsion comprising the polymer matrix and the protein with water to allow particles to form, and recovering the particles.

In another embodiment of the method according to the invention ,solidification is performed by spray-drying.

In another embodiment of the method according to the invention the polymer is a copolymer of mixed D,L-lactic acid and glycolic acid or a copolymer of L-lactic acid and glycolic acid.

In another embodiment of the method according to invention the emulsifier is polyvinyl alcohol or polyvinylpyrrolidone.

In a further embodiment, the stabilizer may be an amino acid such as proline, histidine, glutamine etc., alternatively it may be a sugar such as sucrose or trehalose, or it may also be selected from the group formed by polyols such as mannitol, maltitol or sorbitol, or any combinations of the above.

In another embodiment of the method according to the invention the solvent comprising the polymer matrix is ethyl acetate.

In another embodiment the protein is an antibody.

### BRIEF DESCRIPTION OF THE DRAWINGS

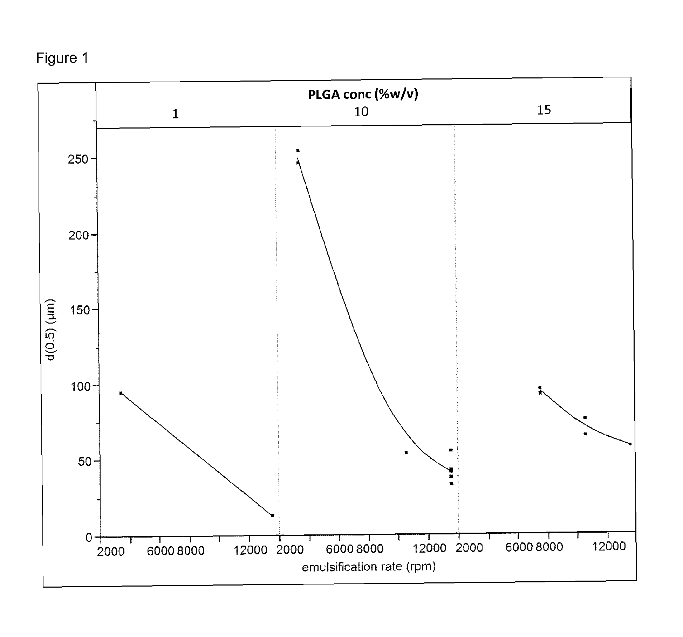

Figure 1: Influence of emulsification rate (from 4500 to 13 500 rpm) on the mean particle size of IgG-loaded PLGA microparticles produced at different PLGA concentrations (1, 10 and 15%) - Figure 1 shows the observed values of particle size versus emulsification rate by PLGA concentration. A smoothed curve (obtained by cubic spline) was added to the graph for clarity

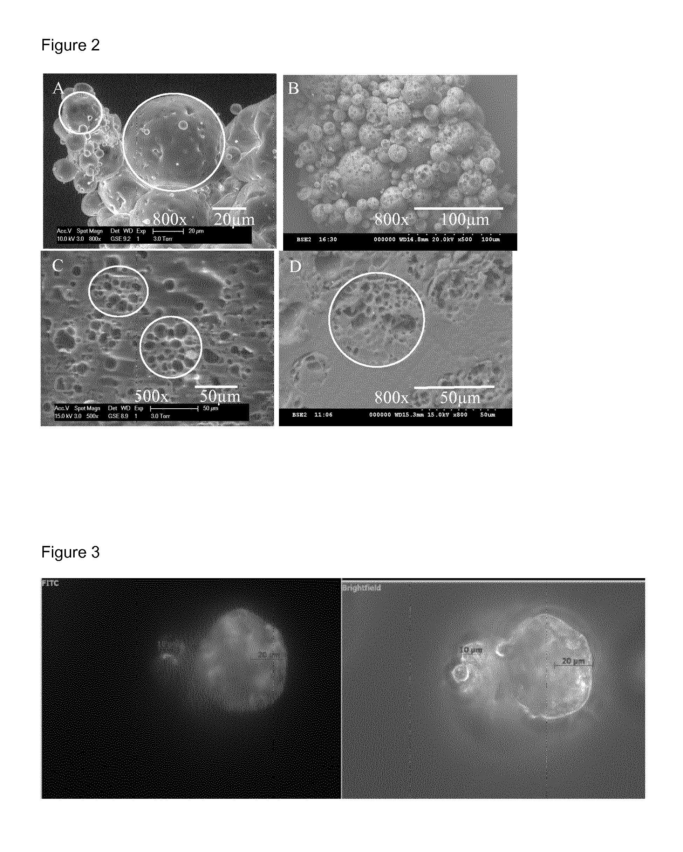

Figure 2: Scanning electron micrographs of IgG-loaded PLGA microparticles: (formulation 25) Surface porosity (a) magnification 800x and(b) magnification 500x and internal morphology after cross-section (c) magnification 500x and (d) magnification 800x; circles added to help visualize the microparticles
Figure 3: Distribution of IgG-FITC loaded PLGA microparticles determined by fluorescence microscopy

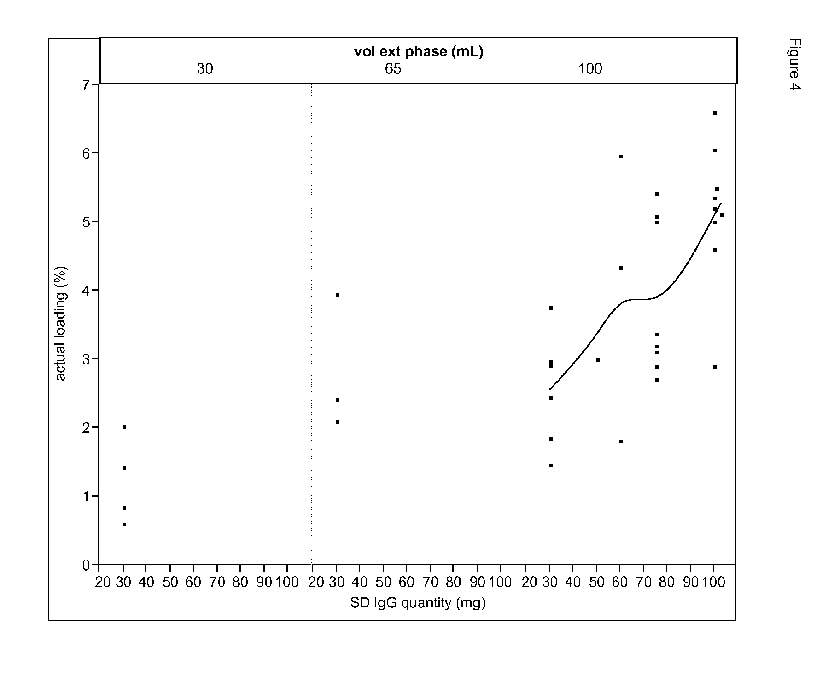

Figure 4: Influence of SD IgG quantities (from 30 to 100 mg) and the volume of the external phase (30, 65 and 100 ml) on the drug loading (% w/w) of IgG-loaded PLGA microparticles - Figure 4 shows the observed values of drug loading versus SD IgG quantities by volume of external phase. A smoothed curve (obtained by cubic spline) was added to the graph for clarity

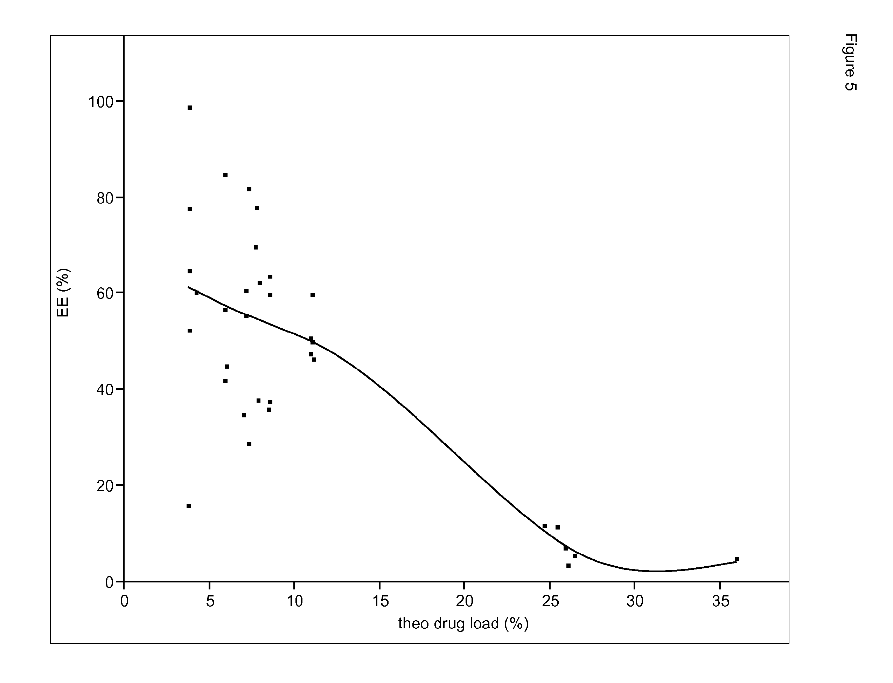

Figure 5: Influence of the theoretical drug load (from 4 to 36% w/w) on the encapsulation efficiency (EE%) of the IgG-loaded PLGA microparticles. Figure 5 shows the observed values of EE% versus theoretical drug load. A smoothed curve (obtained by cubic spline) was added to the graph for clarity

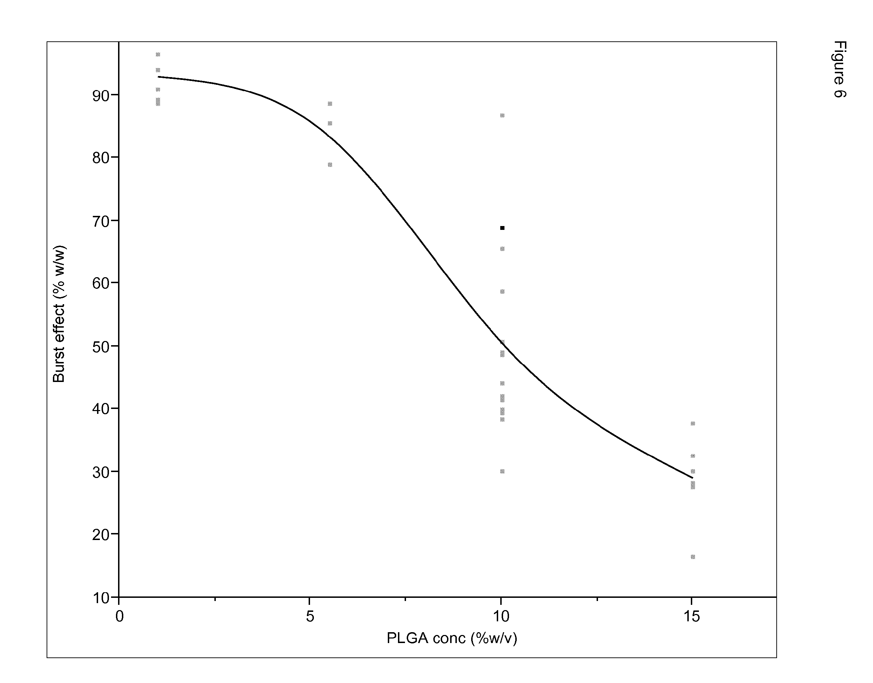

Figure 6: Influence of the PLGA concentrations (from 1 to 15% w/v) on the burst effect (% w/w IgG released after 24h) of IgG-loaded PLGA microparticles - Figure 5 shows the observed values of burst effect versus PLGA concentration. A smoothed curve (obtained by cubic spline) was added to the graph for clarity

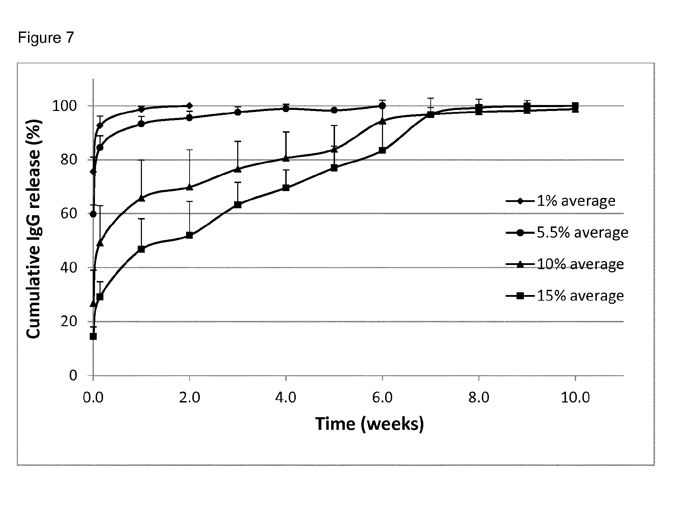

Figure 7: Influence of the PLGA concentration (from 1% to 15% w/v) on the IgG release profile from IgG-loaded PLGA microparticles: (◆) mean dissolution curve of microparticles produced with 1% PLGA solution (n=6), (●) mean dissolution curve of microparticles produced with 5.5% PLGA solution (n=3), (A) mean dissolution curve of microparticles produced with 10% PLGA solution (n=16) and (■) mean dissolution curve of microparticles produced with 15% PLGA solution (n=9)

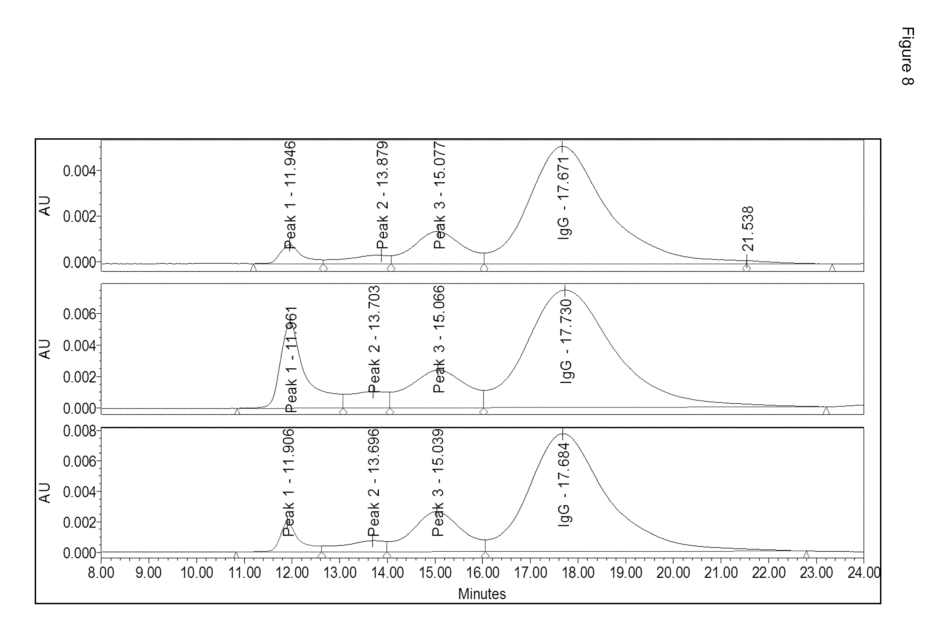

Figure 8: SEC chromatograms of IgG samples eluted as four species: monomer (17.7 min), dimer (15.1 min), trimer (13.7 min) and polyaggregate (11.9 min) - (a) IgG after 1h time point dissolution of 5.5% w/v PLGA microparticles, (b) IgG after 1h time point dissolution of 1% w/v PLGA microparticles, (c) IgG powder in solution (non-encapsulated lyophilized IgG)

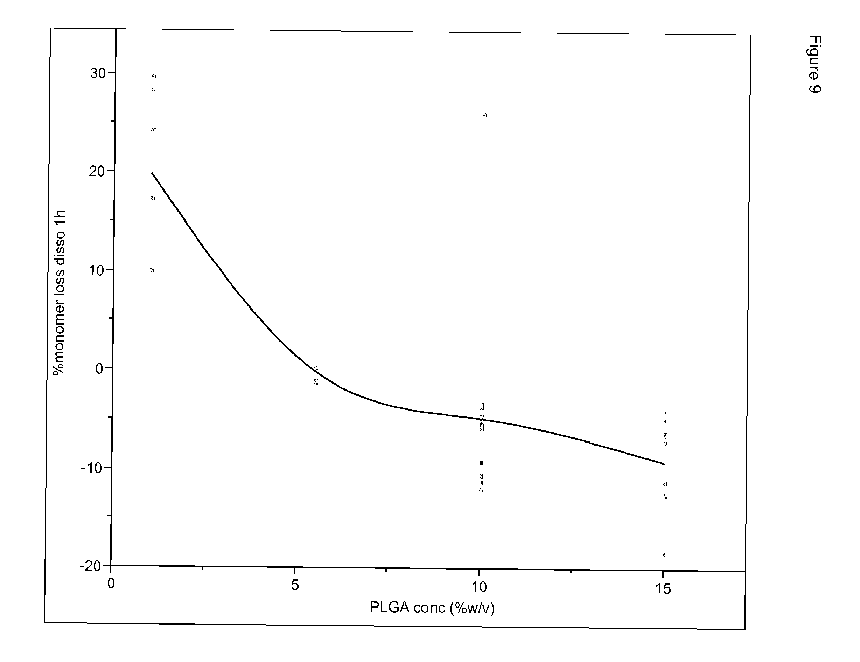

Figure 9: Influence of the PLGA concentrations (from 1 to 15% w/v) on the loss of IgG monomer after 1h dissolution (% w/w) of IgG-loaded PLGA microparticles - Figure 9 shows the observed values of loss of IgG monomer versus PLGA concentration. A smoothed curve (obtained by cubic spline) was added to the graph for clarity

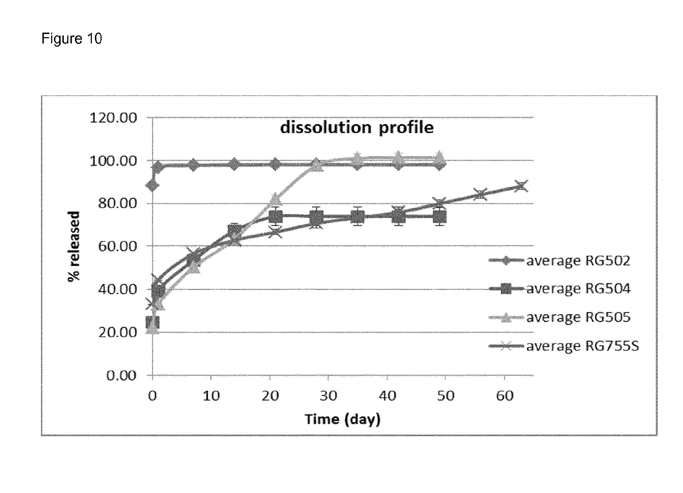

Figure 10: Influence of different typpes of PLGA on the dissolution rate of CDP571-loaded PLGA microparticles. Figure 10 shows the observed percentage of released CDP571.

### DETAILED DESCRIPTION OF THE INVENTION

The present invention addresses the above-identified need by providing a novel method for producing a particle comprising a polymer matrix and a protein. Further provided is a particle comprising a polymer matrix and a protein such as antibody which is suitable for preventive, diagnostic, prognostic or therapeutic applications in animals and humans. The invention also provides a particle comprising a polymer matrix and a protein such as antibody which is suitable for administration to an animal or human by injection, e.g. subcutaneously or intramuscularly.

It has now surprisingly been found by the present inventors that protein denaturation which mainly occurs during formation of water-in-oil (w/o) emulsion in the water-in-oil-in-water (w/o/w) method, can be avoided by use of the solid-in-oil-in-water (s/o/w) method of the present invention for producing a particle comprising a polymer matrix and a protein.

It is an object of the present invention to provide a method for producing a particle comprising a polymer matrix and a protein.

In a first embodiment the invention provides a method for producing a particle comprising a polymer matrix and a protein comprising the steps of solidifying the protein from a solution comprising the protein and stabilizers, combining the solidified protein with a solvent comprising the polymer matrix, combining the solvent comprising the polymer matrix and the protein with an emulsifier in water, agitating the solvent comprising the polymer matrix, the protein and the emulsifier to form an emulsion, combining the emulsion comprising the polymer matrix and the protein with water to allow particles to form, and recovering the particles.

In the second embodiment of the invention protein solidification comprises spray-drying an aqueous solution comprising protein and stabilizers. Alternatively protein solidification according to the present invention comprises protein precipitation from an aqueous solution comprising protein and stabilizers. Said stabilizers may be an amino acid such as proline, histidine, glutamine, etc., alternatively it may be a sugar such as sucrose or trehalose, or it may also be selected from the group formed by polyols such as mannitol, maltitol or sorbitol. Alternatively combinations of any and all of the above types of stabilizers may be used. Stabilizers may be added to the aqueous active protein solution in an amount of between 10 and 50% weight per weight, preferably between 20 and 30% weight of stabilizer per weight of protein. Said aqueous solution typically contains a concentration of protein of between 15 and 75 mg/ml, preferably between 25 and 50 mg/ml.

In the third embodiment of the invention in the method according to the first or second embodiment the polymer is a copolymer of mixed D,L-lactic acid and glycolic acid or a copolymer of L-lactic acid and glycolic acid.

In the fourth embodiment of the invention in the method according to the first, second or third embodiment the ratio of D,L-lactic acid or L-lactic acid to glycolic acid in the copolymer is between 50:50 and 85:15, preferably 75:25.

In the fifth embodiment of the invention in the method according to the first, second third or fourth embodiment the theoretical protein load is not more than 5%, 7%, 8%, 9%, 10%, 12%, 15%, 20% or 25% of weight per weight. Preferably the theoretical protein load is 1% to 25%, 3% to 20%, and most preferably 5% to 15% of weight per weight of total initial particle components, wherein 100% includes collective weight of polymer, surfactant, protein, and stabilizer; also excipients where they are included in the formulation.

In the sixth embodiment of the invention the method according to the first, second, third, fourth or fifth embodiment the solvent is methylene chloride or ethyl acetate.

In the seventh embodiment of the invention in the method according to the first, second, third, fourth, fifth or sixth embodiment the solvent comprises the polymer matrix at a concentration of 1% to 20%, 2% to 17.5% or 5% to 15% weight per volume.

In the eighth embodiment of the invention in the method according to the first, second, third, fourth, fifth, sixth or seventh embodiment the emulsifier is not poly(ethyleneglycol) [PEG].

In the ninth embodiment of the invention in the method according to the first, second third, fourth, fifth, sixth, seventh or eighth embodiment the emulsifier is polyvinyl alcohol or polyvinylpyrrolidone.

In the tenth embodiment of the invention in the method according to the first, second, third, fourth, fifth, sixth, seventh, eighth or ninth embodiment the emulsifier is at a concentration of 0.01% to 10%, 0.025% to 5.0%, 0.05% to 3.0%, 0.1% to 2.5%, 0.1 % to 2.0%, 0.1 % to 2.0% weight per volume in the water.

In the eleventh embodiment of the invention in the method according to the first, second, third, fourth, fifth, sixth, seventh, eighth, ninth or tenth embodiment the volume of water is volume ratio organic solvent:water of between 1:25 to 1:150, preferably 1:26 to 1:100.

In the twelfth embodiment of the invention in the method according to the first, second third, fourth, fifth, sixth, seventh, eighth, ninth, tenth or eleventh embodiment the agitation to form an emulsion is performed by stirring at 3000 rpm to 14000 rpm, 3200 rpm to 13800 rpm, 3400 rpm to 13500 rpm or 5000 rpm to 13500 rpm.

In the thirteenth embodiment of the invention in the method according to the first, second, third, fourth, fifth, sixth, seventh, eighth, ninth, tenth, eleventh or twelfth embodiment the agitation to form an emulsion is performed for at least 5 minutes, at least 20 minutes, at least 30 minutes, at least 1 hour, 2 hours 3 hours or 4 hours. Said agitation to form an emulsion and for subsequent extraction and evaporation of the organic solvent is performed for between 5 minutes and 1 hour, between 5 minutes and 40 minutes, preferably for 30 minutes.

In the fourteenth embodiment of the invention in the method according to the first, second, third, fourth, fifth, sixth, seventh, eighth, ninth, tenth, eleventh, twelfth or thirteenth embodiment the protein is an antibody.

In the fifteenth embodiment of the invention in the method according to the first, second, third, fourth, fifth, sixth, seventh, eighth, ninth, tenth, eleventh, twelfth, thirteenth or fifteenth embodiment the particles are recovered by filtration or centrifugation.

In the sixteenth embodiment of the invention in the method according to the first, second, third, fourth, fifth, sixth, seventh, eighth, ninth, tenth, eleventh, twelfth, thirteenth or fifteenth embodiment the particles are dried under vacuum at 15°C to 35°C, preferably at 20°C to 25°C for 20 minutes to 24 hours following recovery or alternatively they are freeze-dried.

In the seventeenth embodiment of the invention in the method according to the first, second, third, fourth, fifth, sixth, seventh, eighth, ninth, tenth, eleventh, twelfth, thirteenth, fifteenth or sixteenth embodiment wherein the method further comprises attaching a targeting moiety to the particles. Preferably the targeting moiety is selected from among poly(ethyleneglycol) [PEG], poly(L-lysine)-g-poly(ethylene glycol) (PLL-g-PEG), an antibody or a fragment thereof, a receptor targeting peptide, or any suitable combination of the above.

An eighteenth embodiment of the invention is a particle comprising the polymer matrix and a protein obtainable by the method of any of the embodiments.

The nineteenth embodiment of the invention is a particle according to the eighteenth embodiment for use in medicine.

The twentieth embodiment of the invention is a particle according to the eighteenth embodiment for use as a diagnostic.

The twenty-first embodiment of the invention is a pharmaceutical composition comprising the particle according the eighteenth embodiment.

The term "antibody" or "antibodies" as used herein refers to monoclonal or polyclonal antibodies. The term "antibody" or "antibodies" as used herein includes but is not limited to recombinant antibodies that are generated by recombinant technologies as known in the art. "Antibody" or "antibodies" include antibodies' of any species, in particular of mammalian species; such as human antibodies of any isotype, including IgA₁, IgA₂, IgD, IgG₁, IgG₂ₐ, IgG_{2b}, IgG₃, IgG₄ IgE and IgM and modified variants thereof, non-human primate antibodies, e.g. from chimpanzee, baboon, rhesus or cynomolgus monkey; rodent antibodies, e.g. from mouse, rat or rabbit; goat or horse antibodies; and camelid antibodies (e.g. from camels or Ilamas such as Nanobodies™) and derivatives thereof; or of bird species such as chicken antibodies or of fish species such as shark antibodies. The term "antibody" or "antibodies" also refers to "chimeric" antibodies in which a first portion of at least one heavy and/or light chain antibody sequence is from a first species and a second portion of the heavy and/or light chain antibody sequence is from a second species. Chimeric antibodies of interest herein include "primatized" antibodies comprising variable domain antigen-binding sequences derived from a non-human primate (e.g. Old World Monkey, such as baboon, rhesus or cynomolgus monkey) and human constant region sequences. "Humanized" antibodies are chimeric antibodies that contain a sequence derived from non-human antibodies. For the most part, humanized antibodies are human antibodies (recipient antibody) in which residues from a hypervariable region of the recipient are replaced by residues from a hypervariable region [or complementarity determining region (CDR)] of a non-human species (donor antibody) such as mouse, rat, rabbit, chicken or non-human primate, having the desired specificity, affinity, and activity. In most instances residues of the human (recipient) antibody outside of the CDR; i.e. in the framework region (FR), are additionally replaced by corresponding non-human residues. Furthermore, humanized antibodies may comprise residues that are not found in the recipient antibody or in the donor antibody. These modifications are made to further refine antibody performance. Humanization reduces the immunogenicity of non-human antibodies in humans, thus facilitating the application of antibodies to the treatment of human disease. Humanized antibodies and several different technologies to generate them are well known in the art. The term "antibody" or "antibodies" also refers to human antibodies, which can be generated as an alternative to humanization. For example, it is possible to produce transgenic animals (e.g., mice) that are capable, upon immunization, of producing a full repertoire of human antibodies in the absence of production of endogenous murine antibodies. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies with specificity against a particular antigen upon immunization of the transgenic animal carrying the human germ-line immunoglobulin genes with said antigen. Technologies for producing such transgenic animals and technologies for isolating and producing the human antibodies from such transgenic animals are known in the art. Alternatively, in the transgenic animal; e.g. mouse, only the immunoglobulin genes coding for the variable regions of the mouse antibody are replaced with corresponding human variable immunoglobulin gene sequences. The mouse germline immunoglobulin genes coding for the antibody constant regions remain unchanged. In this way, the antibody effector functions in the immune system of the transgenic mouse and consequently the B cell development is essentially unchanged, which may lead to an improved antibody response upon antigenic challenge in vivo. Once the genes coding for a particular antibody of interest have been isolated from such transgenic animals the genes coding for the constant regions can be replaced with human constant region genes in order to obtain a fully human antibody. Other methods for obtaining human antibodies/antibody fragments in vitro are based on display technologies such as phage display or ribosome display technology, wherein recombinant DNA libraries are used that are either generated at least in part artificially or from immunoglobulin variable (V) domain gene repertoires of donors. Phage and ribosome display technologies for generating human antibodies are well known in the art. Human antibodies may also be generated from isolated human B cells that are ex vivo immunized with an antigen of interest and subsequently fused to generate hybridomas which can then be screened for the optimal human antibody. The term "antibody" or "antibodies" as used herein, also refers to an aglycosylated antibody.

The term "antibody" or "antibodies" as used herein also refers to an antibody fragment. A fragment of an antibody comprises at least one heavy or light chain immunoglobulin domain as known in the art and binds to an antigen. Examples of antibody fragments according to the invention include Fab, Fab', F(ab')₂, and Fv and scFv fragments; as well as diabodies; triabodies; tetrabodies; minibodies; domain antibodies; single-chain antibodies; bispecific, trispecific, tetraspecific or multispecific antibodies formed from antibody fragments or antibodies, including but not limited to Fab-Fv constructs. Antibody fragments as defined above are known in the art.

The term "buffer" as used herein, refers to a substance which, by its presence in solution, increases the amount of acid or alkali that must be added to cause unit change in pH. A buffered solution resists changes in pH by the action of its acid-base conjugate components. Buffered solutions for use with biological reagents are generally capable of maintaining a constant concentration of hydrogen ions such that the pH of the solution is within a physiological range. Traditional buffer components include, but are not limited to, organic and inorganic salts, acids and bases.

The term "emulsifier" as used herein is a substance that stabilizes an emulsion by increasing its kinetic stability, this term includes a particular class of emulsifiers known as "surface active substances", or surfactants.

The term "microparticle" as used herein refers to a particle having a diameter of 0.3 to 1000 µm or 0.3 to 700 µm or or 0.7 to 700 µm or 0.3 to 250 µm.

The term "particle" as used herein refers to a small localized object to which can be ascribed several physical properties such as volume or mass, it specifically includes microparticles as defined above. More specifically within the meaning of the present invention, the particle refers to a particle comprising a polymer matrix and a protein.

The term "polymer" as used herein refers to a chemical compound or mixture of compounds consisting of repeating structural units created through a process of polymerization, from which originates a characteristic of high relative molecular mass and attendant properties. Generally, the units composing polymers derive, actually or conceptually, from molecules of low relative molecular mass. For the purposes of drug delivery, thermoplastic aliphatic poly(esters), such as poly-lactide (PLA), poly-glycolide (PGA), and especially PLGA, are useful polymers due to excellent biocompatibility and biodegradability.

The term "matrix" as used herein refers to a three-dimensional structure that may be formed by a polymer. A matrix can entrap or harbor another molecule, such as an active ingredient, in its interior which may, e.g. then be released over extended periods of time.

The term "monoclonal antibody" as used herein refers to a composition of a plurality of individual antibody molecules, wherein each individual antibody molecule is identical at least in the primary amino acid sequence of the heavy and light chains. For the most part, "monoclonal antibodies" are produced by a plurality of cells and are encoded in said cells by the identical combination of immunoglobulin genes. Generally "monoclonal antibodies" are produced by cells that harbor antibody genes, which are derived from a single ancestor B cell.

"Polyclonal antibody" or "polyclonal antibodies", in contrast, refers to a composition of a plurality of individual antibody molecules, wherein the individual antibody molecules are not identical in the primary amino acid sequence of the heavy or light chains. For the most part, "polyclonal antibodies" bind to the same antigen but not necessarily to the same part of the antigen; i.e. antigenic determinant (epitope). Generally, "polyclonal antibodies" are produced by a plurality of cells and are encoded by at least two different combinations of antibody genes in said cells.

The antibody as disclosed herein is directed against an "antigen" of interest. Preferably, the antigen is a biologically important polypeptide and administration of the antibody to a mammal suffering from a disease or disorder can result in a therapeutic benefit in that mammal. However, antibodies directed against non-polypeptide antigens are also contemplated. Where the antigen is a polypeptide, it may be a transmembrane molecule (e.g. receptor) or ligand such as a growth factor or cytokine. Preferred molecular targets for antibodies encompassed by the present invention include CD polypeptides such as CD3, CD4, CD8, CD19, CD20, CD22, CD23, CD30, CD34, CD38, CD40, CD80, CD86, CD95 and CD154; members of the HER receptor family such as the EGF receptor, HER2, HER3 or HER4 receptor; cell adhesion molecules such as LFA-1, Mac1, p150,95, VLA-4, ICAM-1, VCAM and av/b3 integrin including either α or β subunits thereof (e.g. anti-CD11a, anti-CD18 or anti-CD11b antibodies); chemokines and cytokines or their receptors such as IL-1 α and β, IL-2, IL-6, the IL-6 receptor, IL-12, IL-13, IL-17 forms, IL-18, IL-21, IL-23, IL-25, IL-27, TNFα and TNFβ; growth factors such as VEGF; IgE; blood group antigens; flk2/flt3 receptor; obesity (OB) receptor; mpl receptor; CTLA-4; polypeptide C; G-CSF, G-CSF receptor, GM-CSF, GM-CSF receptor, M-CSF, M-CSF receptor; LINGO; BAFF, APRIL; OPG; OX40; OX40-L; and FcRn.

In a further embodiment the invention provides a method for producing a particle comprising a polymer matrix and a protein according to any of the embodiments herein wherein the particle is further modified to attach a targeting moiety. Preferred targeting moieties within the meaning of the present invention include but are not limited to PEG, PLL-g-PEG, antibodies or fragments thereof, receptor binding peptides, or combinations of the above. Methods for adding said targeting moieties may vary depending on the particular moiety to be used, the target tissue or cell and the desired combination of targeting moieties.

In a further embodiment the invention provides a method for treating an animal, a mammal or human subject comprising administering a therapeutically effective amount of the particle or pharmaceutical composition of any of the embodiments disclosed herein to an animal, particularly a mammal, or a human subject wherein the animal, mammal, or human subject, which has a disorder that may be ameliorated through treatment with the particle or pharmaceutical composition, whereby the disorder is cancer, an autoimmune or inflammatory disorder, such as e.g. rheumatoid arthritis, ankylosing spondylitis, inflammatory bowel disease.

For the treatment of the above diseases, the appropriate dosage will vary depending upon, for example, the particular protein or antibody to be employed, the subject treated, the mode of administration and the nature and severity of the condition being treated. Preferred dosage regimen for treating autoimmune diseases and inflammatory disease with the particle of the present invention comprise, for example, the administration of the particle comprising an antibody in an amount of between 1 µg and 1 g.

The particle or pharmaceutical composition according any of the embodiments of the invention is administered preferably by the subcutaneous injection route. The pharmaceutical formulation according to any of the embodiments of the invention may also be administered by intramuscular injection. The pharmaceutical composition may be injected using a syringe or an injection device such as an autoinjector. The pharmaceutical composition to be injected would comprise microparticles at a concentration of between 10 and 40% weight per volume of, preferably between 20 and 30% weight per volume.

As used herein the specification, "a" or "an" may mean one or more. As used herein and in the claim(s), when used in conjunction with the word "comprising", the words "a" or "an" may mean one or more than one.

The use of the term "or" as used herein means "and/or" unless explicitly indicated to refer to alternatives only or the alternatives are mutually exclusive, although the disclosure supports a definition that refers to only alternatives and "and/or."

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

All references cited herein, including journal articles or abstracts, published or unpublished U.S. or foreign patent application, issued U.S. or foreign patents or any other references, are entirely incorporated by reference herein, including all data, tables, figures and text presented in the cited references. Additionally, the entire contents of the references cited within the references cited herein are also entirely incorporated by reference.

### EXAMPLES

### Example 1

### Materials

IgG was used as a model molecule and was purchased as a lyophilized powder from Equitech (Kerrville, USA). PLGA (Resomer^{®} RG504), supplied by Boehringer Ingelheim (Ingelheim, Germany), was used as the biodegradable polymer. Ethyl acetate (EtAc) (Sigma Aldrich, Diegem, Belgium) was used as the organic solvent during the s/o/w encapsulation process. MC (Merck, Darmstadt, Germany) was used to dissolve the PLGA during the encapsulation efficiency evaluation. Polyvinyl alcohol (PVA) - 87-90% hydrolysed - and polyvinylpyrrolidone (PVP) (Sigma-Aldrich, Diegem, Belgium) were used as stabilizers. Mannitol and L-Histidine provided by Sigma Aldrich (Diegem, Belgium) were used to stabilize the IgG during the spray-drying process. Phosphate-buffered saline (PBS) pH 7.2 (Sigma Aldrich, Diegem, Belgium) was employed to buffer the IgG solution. Fluorescein isothiocyanate (FITC), dimethylformamide (DMF), and 50 mM borate buffer, pH 8.5 (Pierce Biotechnology, Rockford, USA) were used to label the IgG. The particles were recovered using nylon filters with a porosity of 0.2 µm (Millipore, Billerica, USA). Amicon 15-30K membranes (Millipore, Billerica, USA) were used to perform the diafiltration.

### Preparation of IgG microparticles using a spray-drying proces

Aqueous IgG solutions, with an IgG concentration of 25 mg/ml in 30% (w/w) mannitol in a 20 mM histidine buffer pH 6.0, were spray-dried using a Mini Spray-dryer B-190 (Büchi Labortechnik, Flawil, Switzerland) and process parameters previously optimized. The inlet temperature (Tin) and the liquid flow rate were set at 130°C and 3 ml/min, respectively. The drying air flow rate was fixed at 30 m³/h and the atomization flow rate at 800 I/h. The resulting outlet temperature (Tout) was 80°C.

### Encapsulation of IgG microparticles in PLGA particles by a s/o/w emulsion process

The IgG was encapsulated using an s/o/w emulsion evaporation/extraction method. Briefly, PLGA was dissolved in 5 ml EtAc under magnetic stirring in a concentration range of 1-15% (w/v) at room temperature. The solid-in-oil (s/o) dispersion was formed by adding 30-150 mg IgG spray-dried powder (SD IgG) into the PLGA organic solution using a T25 digital Ultra-Turrax^{®} high-performance disperser (IK^{®}, Staufen, Germany) set at 13500 rpm. This suspension was added to 30-100 ml of aqueous external phase containing 0.1-2% (w/v) of a surfactant such as PVA or PVP and maintained under agitation using the Ultra-Turrax^{®} stirrer at 3400-13500 rpm. Finally, the s/o/w emulsion was added into an additional volume of water (100-400 ml extraction phase) to produce the final s/o/w emulsion. The resultant emulsion was maintained under magnetic stirring at atmospheric pressure for 30 min to allow both the extraction and the evaporation of the EtAc. As the polymer is insoluble in water, particles containing encapsulated IgG were produced. This is because the rapid extraction of the EtAc in the aqueous phase results in a fast solidification of the polymer. The particles were recovered by filtration, washed several times with Milli Q water and left under vacuum for 48 hours at room temperature.

### Particle size and morphology evaluation

Both the particle size distribution of the IgG:PLGA particles and the spray-dried (SD) IgG microparticles were measured in triplicate using a Malvern Mastersizer Hydro 2000 S (Malvern Instruments, Malvern, UK). SD IgG microparticles were analyzed by laser diffraction after dispersion in isopropanol. A refractive index of 1.52 was used for the IgG microparticles. PLGA particles were analyzed in water as the dispersion medium, using refractive indexes of 1.33 and 1.55 for water and PLGA, respectively. The particle size distribution was evaluated in terms of the median diameter d(0.5) and the d(0.9), which are the diameters below which lay 50% and 90% of the particles, respectively.

Optical microscopy was performed to evaluate the morphology of the produced particles using a Hirox KH-7700 with a Hirox MXG-5040RZ optical system (Hirox Co Ltd, Tokyo, Japan). The particles were re-dispersed in distilled water and placed onto a glass slide.

The morphology of the surface and the internal porosity of the polymeric particles were observed using a scanning electron microscope, model XL30 ESEM-FEG from Philips (Eindhoven, Holland), with an environmental chamber. This microscope was equipped with a field emission gun. The images were obtained using secondary electrons (topographic contrast) at an accelerating voltage of 10 kV. To observe the external surfaces, the particles were deposited on an adhesive conductive support (carbon). Sectional views were made and the internal porosity evaluation was carried out after coating the samples in an epoxy resin and cutting and polishing them with a microtome diamond knife.

### Assessment of drug distribution inside the PLGA particles

The distribution of IgG molecules located in the PLGA particles was studied using the IgG-FITC conjugate and a fluorescence microscope. The IgG labeling was performed using the detailed method described in the Thermo Scientific instruction [18]. Briefly, a 15- to 20-fold molar excess of FITC dissolved in DMF was added to a 5 mg/mL IgG solution (50 mM borate buffer pH 8.5). The excess and hydrolyzed FITC was then removed by buffer exchange using a 10% (w/w) Mannitol in 20 mM histidine buffer of pH 6 and the Amicon^{®} 15-30K centrifugal filter devices after 1 hour of incubation at room temperature in the dark. The IgG-FITC conjugate was then spray-dried and 70 mg of the resulting IgG microparticles were encapsulated using the previously described s/o/w encapsulation method using the optimized process parameters. The drug distribution within the PLGA particles was observed by fluorescence microscopy using a Perkin Elmer LS55.

### IgG assay and stability evaluation

For dissolution study and encapsulation efficiency evaluation, the quantification of the IgG monomer and evaluation of both aggregate and fragment contents were carried out by size exclusion high performance liquid chromatography (SEC) using an HPLC system Agilent 1100 with a UV detector (Agilent Technologies, Waldbronn, Germany). A TSK Gel G3000 SWXL column (Tosoh Bioscience GMBH, Stuttgart, Germany), 7.8 mm ID x 30.0 cm length, with a TSK Gel Guardcol SWXL 5 guard column (Tosoh Bioscience GMBH, Stuttgart, Germany), 6.0 mm ID x 4.0 cm length was used with a 0.5 ml/min flow rate, 20 ml injection volume, and detection at 280 nm. The mobile phase was composed of phosphate buffered saline (PBS) 0.05 M, pH 7.2. The stability of the IgG was evaluated using the percentage of monomer loss that corresponded to the difference in the percentage of monomers before and after the encapsulation step. The concentration of IgG monomer was determined using a calibration curve constructed with known concentrations of the IgG lyophilized powder. The molecular weight of detected species was evaluated using the Bio-Rad Gel Filtration Standard, lyophilized mixture of molecular weight markers ranging from 1350 to 670,000 daltons. (Bio-Rad Laboratories, Hercules, USA).

The total IgG assay was performed using UV spectrophotometry at 280 nm on a Varian^{®} 50 Bio UV/VIS spectrometer equipped with a Solo VPE optical fiber (C Technologies, Inc., Bridgewater, USA).

### Encapsulation efficiency and drug loading evaluation

The drug loading of IgG into the PLGA particles was the ratio between the measured amount of incorporated IgG and the total amount of PLGA and spray-dried IgG that contained both the antibody and inert material. The EE% referred to the amount of IgG detected in the particles compared to the amount of IgG initially introduced into the organic phase. 5-20 mg IgG:PLGA particles were placed in contact with 750 µl of MC to dissolve the polymer. IgG was further extracted using PBS (4 x 750 µl) with 15 min of centrifugation at 8800 rpm. The aqueous phases were collected and analyzed by SE-HPLC for drug loading, EE% and stability evaluations. Complete recovery and absence of degradation were previously checked on non-encapsulated IgG lyophilized powder.

### Dissolution study

Dissolution profiles of IgG from IgG:PLGA particles were evaluated by adding 1 ml of PBS buffer pH 7.2 to 30 mg particles in 2 ml Eppendorf^{®} tubes. The tubes were incubated at 37°C and stirred at 600 rpm using a Thermomixer confort^{®} (Eppendorf AG, Hamburg, Germany). At a pre-determined time, samples were centrifuged for 15 min at 12000 rpm. The supernatant (1 ml) was collected and filtrated on a 0.2 µm HDPE Millex filter (Millipore, England). The particles were suspended in fresh PBS solution. The percentage of released IgG was then measured by SE-HPLC. The burst effect was the percentage of IgG released after a day.

### Design of experiment

The main effects of eight process and formulation variables in the s/o/w encapsulation process were explored. The factors studied were: the PLGA concentration in the organic phase, the stabilizer concentration in the external phase, the volume of the external phase, the s/o/w emulsification time, the s/o/w emulsification speed, the volume of the extraction phase, and the type of external phase stabilizer. The quantity of the spray-dried IgG was then also evaluated. The selected outputs were the particle size, the drug loading, the encapsulation efficiency, the dissolution profile, and the stability of the IgG over the encapsulation process and the dissolution.

A screening design with eight formulations and 3 center replicates of points (from formulation 1 to formulation 11) was constructed using version 8.0.2 JMP statistical software (SAS, NC, USA) to evaluate the main effects of the selected factors. The screening design was then completed with additional formulations (from formulation 12 to formulation 34) to evaluate the second order interactions and the quadratic effects of the PLGA concentration in the organic solvent, the s/o/w emulsification rate, the s/o/w emulsification time, and the quantity of SD IgG dispersed in the organic phase. Mathematical models based on these input-output relationships were constructed both for the screening design and the extended design. The effects of the investigated parameters were determined using the least square method.

### Results

The effects of the studied factors defined in the previous paragraphs were evaluated on different output characteristics. Evaluated formulations are summarized in Table 1 below:

**Table 1: Experimental design - process and formulation parameters**

| **Formulation** | **Block** | **PLGA sol conc. (%w/v)** | **Stab. conc (%w/v)** | **vol extern. (mL)** | **time (min)** | **Rate (rpm)** | **vol extract (mL)** | **Stab.** | **SD IgG quantity (mg)** |
|---|---|---|---|---|---|---|---|---|---|
| 1 | 1 | 5.5 | 1.05 | 65 | 3 | 7025 | 250 | PVA | 30 |
| 2 | 1 | 1 | 2 | 30 | 5 | 3400 | 400 | PVA | 30 |
| 3 | 1 | 5.5 | 1.05 | 65 | 3 | 7025 | 250 | PVA | 30 |
| 4 | 1 | 10 | 0.1 | 30 | 5 | 13500 | 100 | PVA | 30 |
| 5 | 1 | 10 | 2 | 100 | 5 | 13500 | 400 | PVP | 30 |
| 6 | 1 | 10 | 2 | 30 | 1 | 3400 | 100 | PVP | 30 |
| 7 | 1 | 5.5 | 1.05 | 65 | 3 | 7025 | 250 | PVA | 30 |
| 8 | 1 | 10 | 0.1 | 100 | 1 | 3400 | 400 | PVA | 30 |
| 9 | 1 | 1 | 2 | 100 | 1 | 13500 | 100 | PVA | 30 |
| 10 | 1 | 1 | 0.1 | 30 | 1 | 13500 | 400 | PVP | 30 |
| 11 | 1 | 1 | 0.1 | 100 | 5 | 3400 | 100 | PVP | 30 |
| 12 | 1 | 1 | 1 | 100 | 5 | 3400 | 400 | PVA | 60 |
| 13 | 1 | 10 | 1 | 100 | 1 | 13500 | 400 | PVA | 30 |
| 14 | 1 | 1 | 1 | 100 | 5 | 13500 | 400 | PVA | 30 |
| 15 | 1 | 10 | 1 | 100 | 5 | 3400 | 400 | PVA | 60 |
| 16 | 1 | 10 | 1 | 100 | 1 | 3400 | 400 | PVA | 60 |
| 17 | 1 | 15 | 1 | 100 | 3 | 7500 | 400 | PVA | 100 |
| 18 | 1 | 15 | 1 | 100 | 5 | 7500 | 400 | PVA | 50 |
| 19 | 1 | 10 | 1 | 100 | 1 | 13500 | 400 | PVA | 100 |
| 20 | 1 | 15 | 1 | 100 | 1 | 10500 | 400 | PVA | 75 |
| 21 | 1 | 10 | 1 | 100 | 3 | 13500 | 400 | PVA | 75 |
| 22 | 1 | 15 | 1 | 100 | 5 | 13500 | 400 | PVA | 100 |
| 23 | 1 | 10 | 1 | 100 | 5 | 10500 | 400 | PVA | 75 |
| 24 | 1 | 15 | 1 | 100 | 3 | 13500 | 400 | PVA | 75 |
| 25 | 1 | 10 | 1 | 100 | 1 | 13500 | 400 | PVA | 100 |
| 26 | 1 | 10 | 1 | 100 | 1 | 13500 | 400 | PVA | 100 |
| 27 | 2 | 10 | 1 | 100 | 1 | 10500 | 400 | PVA | 75 |
| 28 | 2 | 10 | 1 | 100 | 5 | 13500 | 400 | PVA | 75 |
| 29 | 2 | 10 | 1 | 100 | 1 | 10500 | 400 | PVA | 100 |
| 30 | 2 | 15 | 1 | 100 | 1 | 13500 | 400 | PVA | 100 |
| 31 | 2 | 15 | 1 | 100 | 5 | 10500 | 400 | PVA | 100 |
| 32 | 2 | 15 | 1 | 100 | 1 | 13500 | 400 | PVA | 75 |
| 33 | 2 | 15 | 1 | 100 | 5 | 10500 | 400 | PVA | 75 |
| 34 | 2 | 10 | 1 | 100 | 5 | 13500 | 400 | PVA | 100 |

The results of evaluated formulations are detailed in Table 2 below:

**Table 2: Experimental design - Results of tested formulations**

| **Formulation** | **d(0.5) (µm)** | **d(0.9) (µm)** | **Measured drug loading (%)** | **EE (%)** | **Burst effect (%)** | **Calculated monomer loss after EE% test (%)** | **Calculated monomer loss after 1h disso test (%)** |
|---|---|---|---|---|---|---|---|
| 1 | 37.0 | 65.9 | 4.0 | 55.4 | 88.8 | -5.4 | 0.2 |
| 2 | 28.9 | 56.6 | 1.4 | 5.4 | 94.0 | 5.5 | 24.3 |
| 3 | 34.9 | 74.2 | 2.1 | 28.8 | 79.1 | -5.7 | -1.2 |
| 4 | 23.4 | 42.2 | 0.6 | 15.9 | 86.9 | -2.0 | 26.2 |
| 5 | 15.7 | 51.4 | 2.4 | 64.7 | 68.9 | -12.4 | -9.1 |
| 6 | 255.1 | 794.3 | 2.0 | 52.6 | 44.1 | -7.6 | -4.5 |
| 7 | 38.5 | 65.6 | 2.4 | 35.0 | 85.6 | -8.4 | -1.0 |
| 8 | 193.0 | 387.2 | 3.8 | 98.8 | 30.2 | -8.3 | -9.1 |
| 9 | 15.6 | 28.2 | 2.9 | 11.4 | 96.6 | 3.4 | 9.9 |
| 10 | 8.9 | 45.6 | 0.8 | 11.8 | 96.5 | 33.9 | 29.8 |
| 11 | 95.6 | 215.9 | 1.5 | 3.4 | 90.9 | -8.1 | 10.1 |
| 12 | 64.3 | 365.6 | 1.8 | 5.0 | 88.7 | 4.3 | 28.5 |
| 13 | 37.2 | 92.9 | 3.0 | 77.7 | 41.4 | -2.7 | -5.7 |
| 14 | 13.6 | 144.7 | 1.8 | 7.1 | 89.4 | 0.3 | 17.4 |
| 15 | 246.8 | 625.0 | 6.0 | 81.8 | 50.8 | -3.7 | -4.8 |
| 16 | 159.1 | 356.9 | 4.3 | 60.8 | 65.5 | -1.3 | -5.2 |
| 17 | 96.8 | 235.2 | 5.0 | 62.4 | 30.3 | -2.5 | -7.0 |
| 18 | 93.1 | 221.2 | 3.0 | 60.5 | 28.2 | -3.8 | -6.4 |
| 19 | 43.5 | 118.2 | 4.6 | 50.1 | 58.8 | 0.4 | -3.3 |
| 20 | 65.7 | 144.6 | 2.9 | 41.9 | 28.3 | -1.7 | -11.1 |
| 21 | 38.7 | 120.3 | 3.2 | 37.5 | 40.1 | -0.3 | -3.5 |
| 22 | 58.7 | 164.0 | 2.9 | 37.8 | 37.9 | -1.2 | -4.8 |
| 23 | 53.2 | 128.6 | 3.1 | 36.0 | 42.1 | -1.7 | -5.8 |
| 24 | 60.3 | 184.9 | 2.7 | 45.1 | 28.2 | -2.4 | -6.2 |
| 25 | 34.1 | 86.5 | 5.5 | 50.8 | 50.7 | -2.5 | -3.5 |
| 26 | 33.9 | 90.4 | 5.1 | 46.4 | 50.9 | -1.4 | -3.6 |
| 27 | 55.1 | 110.9 | 5.4 | 63.8 | 39.5 | -7.8 | -12.0 |
| 28 | 55.8 | 110.3 | 5.1 | 59.9 | 38.4 | -7.9 | -11.2 |
| 29 | 54.6 | 113.8 | 6.6 | 59.9 | 49.2 | -7.6 | -10.2 |
| 30 | 58.8 | 141.2 | 6.1 | 78.1 | 32.5 | -8.2 | -12.3 |
| 31 | 66.7 | 145.0 | 5.3 | 69.7 | 32.6 | -7.8 | -18.2 |
| 32 | 54.0 | 132.8 | 5.0 | 84.8 | 27.7 | -8.6 | -12.5 |
| 33 | 76.2 | 180.9 | 3.4 | 56.8 | 16.6 | -9.8 | -4.0 |
| 34 | 42.1 | 94.1 | 5.2 | 47.4 | 48.8 | -6.9 | -10.6 |

### Example 2

In a similar manner to the previous example, microparticle formulations containing monoclonal antibody CDP571 against TNF-alpha were prepared following the same method.

Briefly, microparticles were prepared by the process described above using 10% weight per volume (w/v) concentration of PLGA, 1% (w/v) stabilizer, and an additional external phase of 100 ml, the mixture was emulsified by stirring during 1 minute at 13500 rpm, and an additional extraction volume of 400 ml was used. Varying conditions in terms of stabilizer, buffer and PLGA were assayed. Subsequent freeze-drying process was used to produce CDP571 microparticles.Figure 10 shows an analysis on the influence of varying the type of PLGA used to obtain CDP571 containing microparticle formulations on protein release.

### Reference List

[1] Jain R.A., «The manufacturing techniques of various drug loaded biodegradable poly(lactide-co-glycolide) (PLGA) devices,» Biomaterials 21, pp. 2475-2490, 2000.
[2] M. Muller, J. Voros, G. Csucs, E. Walter, G. Danuser, H. P. Merkle, N. D. Spencer, M. Textor. «Surface modification of PLGA microsphere,s» ; J Biomed Mater Res A. Jul 1;66(1):55-61, 2003.
[3] J. S. Lewis, T. D. Zaveri, C. P. Crooks II, B. G. Keselowsky. «Microparticle surface modifications targeting dendritic cells for non-activating applications,» Biomaterials 33 7221-7232, 2012.
[4] R. Mudargi, V. Ramesh Babu, Vidhya Rangaswany, Pradip Patel et M. A. Tejraj, «Nano/micro technologies for delivery macromolecular therapeutics using poly(D,L-lactide-co-glycolide) and its derivatives,» J. Controlled Release 125, pp. 193-209, 2008.
[5] Y. Mingli, K. Sungwon et P. Kinam, «Issues in long-term protein delivery using biodegradable microparticles,» J. Controlled Release, 2010.
[6] T. Morita, Y. Sakamura, Y. Horikiri, S. Takehiko et H. Yoshino, «Protein encapsulation into biodegradable microspheres by a novel S/O/W emulsion method using poly(ethylene glycol) as a protein micronization adjuvant,» J. Controlled Release 69, pp. 435-444, 2000.
[7] T. Kissel, S. Maretschek, C. Packhauser, J. Schnieders et N. Seidel, «Microencapsulation Techniques for Parenteral Depot Systems and Their Application in the Pharmaceutical Industry,» chez Microencapsulation, Methods and Industrial Applications, Second Edition, vol. 99-122, Edited by Simon Benita, Informa Healthcare, 2005.

## Claims

1. A method for producing a particle comprising a polymer matrix and a protein comprising the steps of:
a) solidifying the protein from a solution comprising the protein,
b) combining the solidified protein with a solvent comprising the polymer matrix,
c) combining the solvent comprising the polymer matrix and the protein with an emulsifier in water,
d) agitating the solvent comprising the polymer matrix, the protein and the emulsifier to form an emulsion,
e) combining the emulsion comprising the polymer matrix and the protein with water to allow particles to form, and
f) recovering the particles.

2. The method according to claim 1 or 2, wherein the polymer is a copolymer of mixed D,L-lactic acid and glycolic acid or a copolymer of L-lactic acid and glycolic acid.

3. The method according to claim 3, wherein the ratio of D,L-lactic acid or L-lactic acid to glycolic acid in the copolymer is 75:25.

4. The method according to any one of claims 1 to 5, wherein the solvent is ethyl acetate.

5. The method according to any one of claims 1 to 6, wherein the solvent comprises the polymer matrix at a concentration of 1 % to 20% weight per volume.

6. The method according to any one of claims 1 to 8, wherein the emulsifier is polyvinyl alcohol.

7. The method according to any one of claims 1 to 9, wherein the emulsifier is at a concentration of 0.05% to 3.0% weight per volume in the water.

8. The method according to any one of claims 1 to 10, wherein the volume of the water is volume ratio organic solvent:water 1:26 to 1:100

9. The method according to any one of claims 1 to 11, wherein the agitation to form an emulsion is performed by stirring at 3400 rpm to 13500 rpm.

10. The method according to any one of claims 1 to 13, wherein the agitation to extract solvent is performed for at least 30 minutes.

11. The method according to any one of claims 1 to 14, wherein the protein is an antibody.

12. The method according to any one of claims 1 to 15, wherein the particles are recovered by filtration.

13. The method according to any one of claims 1 to 16, wherein the particles are dried under vacuum for at least 1 hour following recovery or by freeze-drying.

14. A particle comprising the polymer matrix and a protein obtainable by the method of any one of claims 1 to 17.

15. A pharmaceutical composition comprising the particle according to claim 18.
